# EUROPÄISCHE PATENTSCHRIFT

(11) **EP 0 646 113 B1**
(45) Veröffentlichungstag und Bekanntmachung des Hinweises auf die Patenterteilung: **18.12.1996**
(21) Anmeldenummer: 93912911.0
(22) Anmeldetag: 08.06.1993
(51) Int. Cl.: C07D 401/04, C07D 409/04, C07D 209/52

(54) **SUBSTITUIERTE 3-AZABICYCLO[3.2.0]HEPTAN-DERIVATE ALS ZWISCHENPRODUKTE**
SUBSTITUTED 3-AZABICYCLO[3.2.0]HEPTANE DERIVATES USEFUL AS INTERMEDIATE PRODUCTS
DERIVES SUBSTITUES DE 3-AZABICYCLO[3.2.0]HEPTANE UTILES COMME PRODUITS INTERMEDIAIRES

(30) Priorität: 19.06.1992 DE 4219975
(43) Veröffentlichungstag der Anmeldung: 05.04.1995
(73) Patentinhaber: BASF Aktiengesellschaft, 67063 Ludwigshafen (DE)
(72) Erfinder: STEINER, Gerd, D-6719 Kirchheim (DE)
(86) Internationale Anmeldenummer: EP9301439
(87) Internationale Veröffentlichungsnummer: WO9400445

(56) Entgegenhaltungen:
- WO-A-94/00431
- WO-A-94/00458
- HELVETICA CHIMICA ACTA, Bd. 59, Nr. 4, 1976, BASEL, CH; Seiten 1186 - 1202, W. OPPOLZER et al.: "Stereoselective syntheses of benz(f)isoindoline-derivatives by intramolecular cycloadditions of styrenes to olefins"

## Beschreibung

Die Erfindung betrifft neue Azabicycloheptan-Derivate, deren Herstellung und Verwendung zur Herstellung von Pharmawirkstoffen.

In Helv. Chim. Acta 59, 1186 (1976) sind 3-Azabicyclo[3.2.0]heptan-Derivate beschrieben, die durch Thermolyse hergestellt werden.

Gegenstand der Erfindung sind 3-Azabicyclo[3.2.0]heptan-Derivate der Formel I 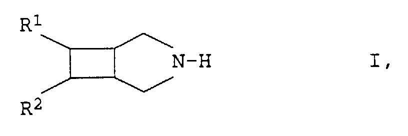 worin
- R¹: eine gegebenenfalls durch Halogenatome, C₁-C₄-Alkyl-, Trifluormethyl-, C₁-C₄-Alkoxy-, Hydroxy-, Amino-, Monomethylamino-, Dimethylamino-, Cyano- oder Nitrogruppen mono- oder disubstituierte Phenyl-, Pyridyl-, Thienyl- oder Pyrrolgruppe bedeutet und
- R²: ein Wasserstoffatom oder eine gegebenenfalls durch Fluor, Methoxy, Hydroxy oder Amino substituierte Phenylgruppe ist.

Bevorzugt sind die Verbindungen der Formel I, in denen R² ein Wasserstoffatom und R¹ einen gegebenenfalls durch Fluor, Chlor, Hydroxy, Methoxy oder Amino substituierten Phenylrest darstellen. Die Phenylgruppe steht am Bicyclus bevorzugt in der exo-Konfiguration.

Die Verbindungen der Formel I können auch als Salze vorliegen.

Die neuen Verbindungen lassen sich herstellen, indem man ein Amin der Formel II 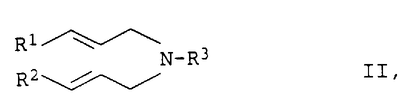 worin R¹ und R² die oben angegebenen Bedeutungen besitzen und R³ ein Wasserstoffatom oder eine Acetyl-, Trifluoracetyl- oder Benzylgruppe ist, in einem inerten Lösungsmittel bei einer Temperatur von 20-80°C in Gegenwart von etwa 1 Mol Salzsäure pro Mol Amin photochemisch einer 2+2 Cycloaddition unterwirft und eine gegebenenfalls vorhandene Acyl- oder Benzylgruppe für R³ abspaltet.

Die Photoreaktion gelingt gut in Aceton. Als Lichtquelle eignet sich besonders gut eine Quecksilberhochdrucklampe. Es kann vorteilhaft sein, die Photocycloaddition in einer Quarzapparatur unter einer Stickstoffatmosphäre durchzuführen.

Die Photocycloaddition verläuft - wie die Röntgenstruknuranalyse zeigt - in den meisten Fällen hochdiastereoselektiv zu den bicyclischen Verbindungen I mit der exo-Konfiguration bezüglich R¹ und R².

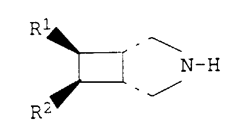

Durch Racematspaltung, z.B. mit optisch aktiven Weinsäure-Derivaten, lassen sich die beiden Enantiomeren rein isolieren.

Die Amine der Formel II sind literaturbekannt oder lassen sich herstellen, indem man entweder einen Aldehyd R¹-CHO mit Vinyl-magnesiumchlorid zum Allylalkohol III 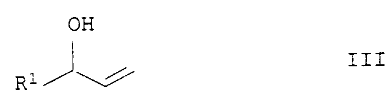 umsetzt, anschließend mit Chlorwasserstoff zum Allylchlorid IV 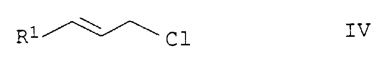 umlagert und zuletzt mit dem entsprechenden Allylamin V 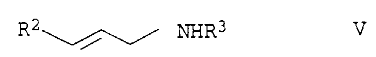 substituiert, oder man unterzieht einen Zimtaldehyd VI 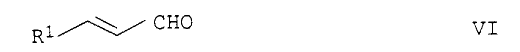 direkt der reduktiven Aminierung mit dem Allylamin V.

Die Abspaltung eines Acylrestes aus den Verbindungen II erfolgt zweckmäßig durch Verseifung nach bekannten Methoden. Analoges gilt für die Abspaltung des Benzylrestes.

Die erfindungsgemäßen Verbindungen der Formel I sind wertvolle Zwischenprodukte zur Synthese neuer pharmakologisch wirksamer Verbindungen der Formeln VII (vgl. WO 94/00431) und VIII (vgl. WO 94/00458) 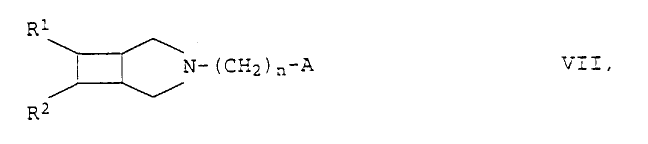 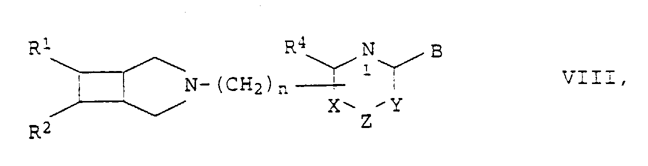 worin
- R¹ und R²: die angegebenen Bedeutungen haben,
- n: die Zahl 1, 2, 3 oder 4 bedeutet,
- A: ein Wasserstoffatom oder einer der Reste 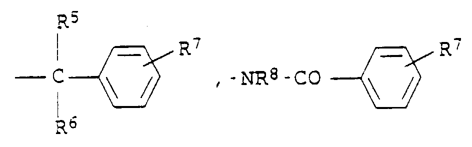 oder -CH=CH2 ist, worin
- R⁵: ein Wasserstoffatom, einen Hydroxyrest und einen gegebenenfalls durch ein Fluor-, Chlor- oder Bromatom substituierten Phenylrest darstellt,
- R⁶: ein Wasserstoffatom bedeutet oder
- R⁵ und R⁶: zusammen ein Sauerstoffatom darstellen,
- R⁷: ein Wasserstoff-, Fluor-, Chlor- oder Bromatom oder eine Hydroxyl-, Nitro-, C₁-C₄-Alkyl- oder Methoxygruppe bedeuten,
- R⁸: ein Wasserstoffatom oder eine Methylgruppe darstellt,
- R⁴: ein Wasserstoffatom, eine Hydroxy-, C₁-C₄-Alkyl- oder C₁-C₄-Alkoxy-Gruppe ist, cder zusammen mit dem benachbarten Kohlenstoffatom eine C=O- oder C=S-Gruppe bedeutet,
- X und Y: Kohlenstoffatome, CH-, CH₂-, NH- oder C₁-C₄-Alkyl-N-Gruppen oder Stickstoffatome bedeuten,
- Z: eine direkte Bindung, eine CO-Gruppe, CS-Gruppe oder eine CH- bzw. CH₂-Gruppe, in denen ein Wasserstoffatom gegen eine Hydroxy-, Amino- oder C₁-C₄-Alkoxygruppe oder ein Halogenatom ausgetauscht sein kann, darstellt, und
- B: ein Wasserstoffatom, eine Hydroxy-, Amino-, Mercapto-, C₁-C₄-Alkylamino-, Di-C₁-C₄-Alkylamino-, C₁-C₄-Alkylthio- oder C₁-C₄-Alkoxygruppe oder zusammen mit dem benachbarten Kohlenstoffatom eine C=O-Gruppe bedeutet, oder
- B: eine mit Y verknüpfte C₃-C₄-Alkylengruppe bedeutet, die eine oder zwei nicht kumulierte Doppelbindungen enthalten kann und in der eine CH- oder CH₂-Gruppe durch ein Stickstoff- oder Schwefelatom oder eine NH- oder N-CH₃-Gruppe ersetzt sein kann und wobei der Ring entweder durch ein Fluor- oder Chloratom oder eine Methyl-, Methoxy-, Nitro- oder Aminogruppe monosubstituiert oder im Fall eines Benzolrings dieser durch Fluor- oder Chloratome oder Methyl-, Trifluormethyl-, Nitro-, Hydroxy-, Methoxy-, Amino-, Monomethyl- oder Dimethylaminogruppen mono-, di- oder trisubstituiert sein kann,
und worin der Ring im rechten Teil der Formel VIII am Stickstoffatom Nr. 1 eine C₁-C₄-Alkyl-, Allyl- oder Benzylgruppe tragen und 1 bis 3 nicht kumulierte Doppelbindungen enthalten kann,
und deren Salze mit physiologisch verträglichen Säuren.

Die Verbindungen der Formeln VII und VIII erhält man aus den Verbindungen I durch Umsetzen mit Verbindungen der Formeln IX und X

Nu-(CH₂)ₙ-A IX,

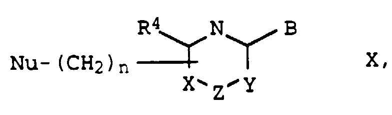

worin Nu eine nukleofuge Abgangsgruppe, beispielsweise ein Halogenatom, insbesondere ein Brom- oder Chloratom, darstellt und A, B, X, Y, Z, R⁴ und n die oben angegebene Bedeutung haben. Die Umsetzung findet zweckmäßig in einem inerten Lösungsmittel wie Tetrahydrofuran, Toluol oder Xylol in Gegenwart einer inerten Base wie Kaliumcarbonat oder Triethylamin bei 80 bis 150°C statt.

Die Verbindungen der Formeln VII und VIII besitzen wertvolle pharmakologische Eigenschaften, beispielsweise als Neuroleptika, Antidepressiva, Sedativa, Hypnotika, ZNS-Protektiva oder Muskelrelaxantien.

Die folgenden Beispiele dienen zur Erläuterung der Erfindung.

### A Herstellung der Ausgangsmaterialien

### 1. N-Cinnamyl-N-allylamin

Zu 173 ml (1,16 M) Cinnamylchlorid in 1350 ml Tetrahydrofuran fügte man 175 ml (1,27 M) Triethylamin hinzu, erwärmte auf 60°C und tropfte anschließend 105 ml (1,40 M) Allylamin zu. Man ließ den Reaktionsansatz 6 h am Rückfluß kochen und rührte dann noch 10 h bei Raumtemperatur nach. Nach Einengen im Wasserstrahlvakuum verteilte man den Kolbeninhalt zwischen Methyl-t-butyl-ether und Wasser (pH = 10). Man extrahierte die wäßrige Phase noch zweimal mit Methyl-t-butyl-ether nach und schüttelte anschließend die vereinigten organischen Phasen mit einer Mischung von 250 ml konzentrierter Salzsäure in 2 1 Wasser durch. Dann extrahierte man die salzsaure wäßrige Phase mit 1,2 1 Methylenchlorid und wusch die Methylenchloridphase noch fünfmal mit je 500 ml 10 %iger Salzsäure nach. Die vereinigten salzsauren wäßrigen Phasen stellte man mit konzentrierter Natronlauge alkalisch und extrahierte zweimal mit Methylenchlorid. Nach Trocknen und Einengen der organischen Phase erhielt man 66,5 g Rohprodukt, das bei 86 bis 88°C im Ölpumpenvakuum (0,25 mbar) destilliert wurde. Ausbeute: 64 g (32 %).

### 2. N-Cinnamyl-benzylamin und Bis-(N-cinnamyl)-benzylamin

Zu 96,4 g (600 mM) Cinnamylchlorid in 800 ml Tetrahydrofuran fügte man 113 ml (818 mM) Triethylamin hinzu, erwärmte auf 60°C und tropfte anschließend 65,4 ml (600 mM) Benzylamin zu. Man ließ den Reaktionsansatz 3 h am Rückfluß kochen und rührte dann noch 15 h bei Raumtemperatur nach. Nach Einengen im Wasserstrahlvakuum verteilte man den Kolbeninhalt zwischen Methyl-t-butylether und Wasser (pH = 10). Man wusch die organische Phase noch zweimal mit Wasser, trocknete mit Natriumsulfat und engte ein. Das Rohprodukt (109 g) aus Mono- und Bisaddukt löste man in 400 ml Aceton, fügte eine Lösung aus 48 g (413 mM) Maleinsäure in 250 ml Aceton unter Rühren hinzu und saugte nach dem Kühlen die ausgefallenen Festkörper ab. Nach dem Waschen mit Aceton isolierte man 60,0 g (30 %) N-Cinnamyl-benzylamin als Maleinat, Schmp.: 169 - 170°C.

Die Mutterlauge engte man vollständig ein und rührte den Rückstand mit Essigester über Nacht aus. Die ausgefallenen Festkörper saugte man nach Eiskühlung ab und wusch mit Essigester nach. Man isolierte 65,0 g (48 %) Bis-(N-cinnamyl)-benzylamin als Maleinat, Öl.

### 3. N-Cinnamyl-N-allyl-benzylamin

Zu 10,0 g (44,8 mM) N-Cinnamyl-benzylamin in 60 ml Ethanol wurden 11,0 g (90 mM) Allylbromid und anschließend 5,0 g (50,0 mM) Triethylamin unter Rühren gegeben. Man ließ den Reaktionsansatz 4 h am Rückfluß kochen. Nach Einengen im Wasserstrahlvakuum verteilte man den Kolbeninhalt zwischen Methylenchlorid und Wasser, stellte mit verdünnter Natronlauge alkalisch und extrahierte noch zweimal mit Methylenchlorid nach. Die vereinigten organischen Phasen wusch man mit verdünnter Natronlauge nach, trocknete und engte ein. Die Reinigung des Rohproduktes (12,5 g) erfolgte durch Säulenchromatographie (Kieselgel, Laufmittel Methylenchlorid/Methanol 98/2). Man erhielt 9,2 g (80 %) Produkt (Öl).

### 4. 1-(4-Fluorphenyl)allylalkohol

In einen 4-1-Rührkolben wurden unter Stickstoff 1550 ml (2,0 M) einer 1,29 M Lösung von Vinylmagnesiumchlorid in Tetrahydrofuran gefüllt. Anschließend wurde unter Rühren und unter Stickstoff innerhalb von 30 min bei 30 - 35°C eine Lösung von 222,0 g (1,764 M) 4-Fluorbenzaldehyd in 2000 ml Tetrahydrofuran zugegeben, wobei der Reaktionsansatz mit Eis gekühlt wurde. Das Reaktionsgemisch wurde noch 2,5 h unter Stickstoff bei Raumtemperatur gerührt. Danach wurde unter Rühren und Kühlen mit Eis 180 ml Wasser.zugesetzt, abgesaugt und der Filterrückstand 3x mit 150 ml Tetrahydrofuran gewaschen. Die Filtrate wurden vereinigt, mit Natriumsulfat getrocknet und eingeengt. Man erhielt 265,7 g (99 %) Produkt in Form eines gelbbraunen Öls.

### 5. 3-(4-Fluorphenyl)allylchlorid

273,6 g (1,798 M) 1-(4-Fluorphenyl)allylalkohol wurden unter Rühren in 2000 ml Methylenchlorid gelöst. Anschließend wurden in 3 h 101,0 g (2,770 M) Chlorwasserstoff eingeleitet, wobei die Temperatur auf bis zu 37°C stieg. Es wurde 1 h nachgerührt. Nach dem Waschen mit 600 ml eiskaltem Wasser und einer Mischung aus 150 ml gesättigter Kochsalzlösung und 150 ml Wasser wurde die organische Phase über Natriumsulfat getrocknet und eingeengt. Man erhielt 294,6 g (98 %) eines braunen Öls.

### 6. N-Allyl-N-[3-(4-fluorphenyl)allyl]amin

Zu einer Lösung von 795,0 g (13,92 M) Allylamin in 360 ml Toluol wurden unter Rückfluß innerhalb von 25 min 231,8 g (1,359 M) 3-(4-Fluorphenyl)allylchlorid zugegeben und noch 1 h bei der Rückflußtemperatur nachgerührt. Anschließend wurde über eine 10 cm-Destillationskolonne (5 mm Glasringe) bei einer Badtemperatur von bis zu 125°C 1000 ml abdestilliert. Der Destillationsrückstand wurde mit 1000 ml Wasser versetzt, mit 38 %iger Salzsäure auf pH 0,7 eingestellt. Die organische Phase wurde abgetrennt und verworfen. Die wäßrige Phase wurde mit 50 %iger Natronlauge auf pH 12,7 eingestellt und mit Toluol extrahiert. Die Toluolextrakte wurden mit Natriumsulfat getrocknet und eingeengt. Der Rückstand wurde über eine Kolonne bei 0,7 bis 1 mbar destilliert. Bei einer Badtemperatur von 120 - 160°C erhielt man 191,8 g (74 %) eines hellgelben Öls.

### 7. N-Allyl-N-3-(3,5-dichlorphenyl)allylamin

Zu 12,0 g (59,7 mM) 3,5-Dichlorzimtaldehyd in 180 ml Methylenchlorid wurden 4,5 ml (60 mM, 3,4 g) Allylamin sowie 17,0 g Natriumsulfat gegeben und die Reaktionsmischung 24 h bei Raumtemperatur gerührt. Danach wurde das Natriumsulfat abfiltriert, mit Methylenchlorid nachgewaschen und das Filtrat zur Trochene eingeengt. Das so erhaltene gelbe Öl wurde in 200 ml absolutem Methanol gelöst, und unter Stickstoff wurde portionsweise 2,5 g (66,0 mM) Natriumborhydrid zugegeben. Die sich leicht erwärmende Reaktionsmischung wurde noch 1 h nachgerührt und danach mit 10 %iger Salzsäure neutralisiert (pH = 7). Das Lösungsmittel wurde im Vakuum entfernt und der verbleibende Rückstand in Methylenchlorid aufgenommen. Die organische Phase wurde zweimal mit Wasser gewaschen, über Natriumsulfat getrocknet und eingeengt. Der verbleibende Rückstand wurde mittels Säulenchromatographie (Kieselgel, Methylenchlorid + 5 % Methanol) gereinigt. Ausbeute: 9,2 g (63 %) gelbes Öl.

### 8. N-Allyl-2,2,2-trifluoro-N-[3-(3-pyridyl)-allyl]-acetamid

Zu einer Lösung von 10,0 g (57,5 mM) N-Allyl-N-3-(3-pyridyl)allylamin und 10,7 ml Triethylamin in 100 ml Tetrahydrofuran wurden bei 0°C 16,1 g (76,6 mM) Trifluoracetanhydrid langsam zugetropft. Es wurde noch 2 h bei Raumtemperatur nachgerührt. Danach wurde die Reaktionslösung auf 250 ml Eiswasser gegossen und dreimal mit je 150 ml Methyl-tert.-butylether extrahiert. Die vereinigten organischen Phasen wurden über Natriumsulfat getrocknet und eingeengt: 14,3 g (92 %) dunkelbraunes Öl.

### B Herstellung der Endprodukte

### 1. exo-6-(p-Fluor)- phenyl-3-azabicyclo[3.2.0]heptan

19,4 g (102 mM) N-Allyl-N-[3-(4-fluorphenyl)allyl]amin in 130 ml Aceton wurden mit 130 ml 10 %iger Salzsäure sowie mit 600 mg Michlers Keton versetzt und unter Stickstoff 55 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Ausbeute 19,3 g (99 %), Schmp. 165-166°C (Maleinat).

Zur Trennung der Antipoden versetzte man 15,0 g (78,5 mM) des Racemats mit einer Lösung von 31,7 g (78,5 mM) (-)-Di-O-toluoyl-L-weinsäure in 300 ml siedendem Ethanol. Die beim Abkühlen unter Rühren ausfallenden Kristalle (13,8 g) wurden unter Nachwaschen mit Ethanol abgesaugt und aus 300 ml Ethanol unter Zusatz von 200 ml Wasser umkristallisiert. Freisetzen der Base lieferte den (+)-Antipoden (5,5 g) mit [α]_{D} = + 97,0° (EtOH, c = 0,969).

Aus der obigen Mutterlauge kristallisierten über Nacht 14,2 g eines Salzes, das aus 400 ml Ethanol (Abfiltrieren des unlöslichen Anteils in der Siedehitze) umkristallisiert wurde (Einmengen auf 300 ml). Freisetzen der Base ergab 4,0 g des (-)-Antipoden mit [α]_{D} = - 96,0° (EtOH, c = 0,940).

Die exo-Phenyl-Konfigurationen wurden mittels Röntgenstrukturanalyse nachgewiesen.

### 2. exo-6-Phenyl-3-azabicyclo[3.2.0]heptan

50,0 g (28,9 mM) N-Cinnamyl-N-allylamin in 1600 ml Aceton wurden mit 300 ml 10 %iger Salzsäure versetzt und unter Stickstoff 48 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Ausbeute 49,0 g (98 %) viskoses Öl, Schmp. 177 bis 178°C (Maleinat).

### 3. exo-6,7-Diphenyl-3-benzyl-3-azabicyclo[3.2.0]heptan

70,0 g (206 mM) Bis-(N-Cinnamyl)-benzylamin in 2500 ml Aceton wurden mit 0,8 g Michlers Keton versetzt und unter Stickstoff 25 h mit einer 150 Watt Quecksilber-Hochdrucklampe in einer Duranglasapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Die Reinigung des Rohproduktes (65,0 g) erfolgte durch Säulenchromatographie (Kieselgel, Laufmittel Toluol/Ethanol 98/2). Man erhielt 58,0 g (83 %) Produkt, Schmp. 230-232°C (Hydrochlorid).

### 4. exo-6,7-Diphenyl-3-azabicyclo[3.2.0]heptan

Zu 12,0 g (35,4 mM) exo-6,7-Diphenyl-3-benzyl-3-azabicyclo[3.2.0]heptan in einer Mischung aus 300 ml n-Propanol und 16 ml Wasser wurden 16,0 g (254 mM) Ammoniumformiat sowie 2,0 g Palladium (10 %ig) auf Kohle gegeben und die Reaktionsmischung 4 h am Rückfluß gekocht (Entwicklung von Kohlendioxid). Nach dem Abkühlen saugte man vom Katalysator ab, wusch mit Propanol und Methylenchlorid nach und engte das Filtrat ein. Man verteilte den Rückstand zwischen Methylenchlorid und Wasser, stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Man erhielt 8,1 g (92 %) Produkt, Schmp. 140 bis 142°C (Maleinat).

### 5. exo-6-Phenyl-3-benzyl-3-azabicyclo[3.2.0]heptan

9,2 g (35,0 mM) N-Cinnamyl-N-allyl-benzylamin in 1100 ml Aceton wurden mit 100 mg Michlers Keton versetzt und unter Stickstoff 5 h mit einer 150 Watt Quecksilber-Hochdrucklampe in einer Duranglasapparatur bei Raumtemperatur bestrahlt. Anschließend engte man den Reaktionsansatz ein. Die Reinigung des Rohproduktes (9,4 g) erfolgte durch Säulenchromatographie (Kieselgel, Laufmittel Methylenchlorid/Methanol 98/2). Man erhielt 3,3 g (36 %) Produkt, Schmp. 126-128°C (Maleinat).

### 6. 2,2,2-Trifluoro-l-[exo-6-(3-pyridyl)-3-azabicyclo[3.2.0]hept-3-yl]-ethanon

14,0 g (51,8 mM) N-Allyl-2,2,2-trifluoro-N-[3-(3-pyridyl)-allyl]-acetamid wurden in 140 ml Aceton gelöst, mit 30 ml 10 %iger wäßriger Salzsäure versetzt und unter Stickstoff 48 h mit einer 150 Watt Quecksilber-Hochdrucklampe in einer Duranglasapparatur bei Raumtemperatur bestrahlt. Danach wurde die Reaktionslösung eingeengt, in 150 ml Wasser aufgenommen und mit wäßriger Ammoniaklösung auf pH 8-9 eingestellt. Die wäßrige Phase wurde zweimal mit tert.-Butylmethylether extrahiert, die vereinigten organischen Phasen über Natriumsulfat getrocknet und eingeengt. Der verbleibende Rückstand wurde mittels Säulenchromatographie (Kieselgel, Methylenchlorid + 2 % Methanol) fraktioniert. Man erhielt 6,2 g (42 %) unverändertes N-Allyl-2,2,2-trifluoro-N-[3-(3-pyridyl)allyl]-acetamid und 3,7 g (26 %) 2,2,2-Trifluoro-1-[exo-6-(3-pyridyl)-3-azabicyclo[3.2.0]hept-3-yl]-ethanon als dunkles Öl.

### 7. exo-6-(3-Pyridyl)-3-azabicyclo[3.2.0)heptan

Zur Lösung von 3,7 g (13,7 mM) 2,2,2-Trifluoro-1-[exo-6-(3-pyridyl)-3-azabicyclo[3.2.0]hept-3-yl]-ethanon in 50 ml Ethanol wurden 2,5 g Kaliumhydroxid-Plätzchen gegeben. Die Reaktionslösung wurde noch 2 h bei Raumtemperatur nachgerührt und anschließend auf 100 ml Eiswasser gegossen. Die wäßrige Phase wurde dreimal mit tert.-Butyl-methylether extrahiert, die vereinigten organischen Phasen wurden über Natriumsulfat getrocknet und eingeengt. Ausbeute 2,3 g (96 %) gelbes Öl, Schmp. 202-205°C (Hydrochlorid).

Analog lassen sich folgende Endprodukte herstellen:
8. exo-6-(m-Fluor-phenyl)-3-azabicyclo[3.2.0]heptan
9. exo-6-(o-Fluor-phenyl)-3-azabicyclo[3.2.0]heptan, Schmp. 118-120°C (Maleinat)
10. exo-6-(p-Chlor-phenyl)-3-azabicyclo[3.2.0]heptan, Schmp. 152-154°C (Maleinat)
11. exo-6-(m-Chlor-phenyl)-3-azabicyclo[3.2.0]heptan Schmp. 130-132°C (Maleinat)
12. exo-6-(p-Methoxy-phenyl)-3-azabicyclo[3.2.0]heptan
13. exo-6-(m-Methoxy-phenyl)-3-azabicyclo[3.2.0]heptan
14. exo-6-(p-Nitro-phenyl)-3-azabicyclo[3.2.0]heptan Schmp. 158-160°C (Maleinat)
15. exo-6-(m-Nitro-phenyl)-3-azabicyclo[3.2.0]heptan
16. exo-6-(p-Trifluormethyl-phenyl)-3-azabicyclo-[3.2.0]heptan, Schmp. 155-156°C (Maleinat)
17. exo-6-(m-Trifluormethyl-phenyl)-3-azabicyclo-[3.2.0]heptan
18. exo-6-(3,4-Dichlor-phenyl)-3-azabicyclo-[3.2.0]-heptan
19. exo-6-(3,5-Dichlor-phenyl)-3-azabicyclo-[3.2.0]-heptan, Schmp. > 250°C (Hydrochlorid)
20. exo-6-(3,4-Dimethoxy-phenyl))-3-azabicyclo-[3.2.0]-heptan
21. exo-6-(m-Hydroxy-phenyl)-3-azabicyclo-[3.2.0]heptan
22. exo-6-(p-Hydroxy-phenyl)-3-azabicyclo-[3.2.0]heptan
23. exo-6-(3,4-Dihydroxy-phenyl)-3-azabicyclo-[3.2.0]-heptan
24. exo-6-(p-Methyl-phenyl)-3-azabicyclo-[3.2.0]heptan
25. exo-6-(m-Methyl-phenyl)-3-azabicyclo-[3.2.0]heptan
26. exo-6-(p-t-Butyl-phenyl)-3-azabicyclo-[3.2.0]heptan, Schmp. > 255°C (Hydrochlorid)
27. exo-6-(m-Amino-phenyl)-3-azabicyclo-[3.2.0]heptan
28. exo-6-(p-Amino-phenyl)-3-azabicyclo-[3.2.0]heptan
29. exo-6-(p-Cyano-phenyl)-3-azabicyclo-[3.2.0]heptan, Schmp. 168-170°C (Maleinat)
30. exo-6-Thien-2-yl-3-azabicyclo-[3.2.0]heptan, Schmp. 180-182°C (Hydrochlorid)
31. exo-6-Thien-3-yl-3-azabicyclo-[3.2.0]heptan, Schmp. 143-145°C (Hydrochlorid)
32. exo-6- (5-Chlor-thien-2-yl)-3-azabicyclo-[3.2.0]-heptan, Schmp. 156-157°C (Maleinat)
33. exo-6-Pyrrol-2-yl-3-azabicyclo-[3.2.0]heptan
34. exo-6-Pyrid-4-yl-3-azabicyclo-[3.2.0]heptan
35. exo-6-Pyrid-2-yl-3-azabicyclo-[3.2.0]heptan

## Patentansprüche

1. 3-Azabicyclo[3.2.0]heptan-Derivate der Formel I 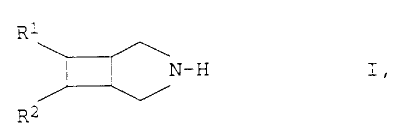 worin
R¹ eine gegebenenfalls durch Halogenatome C₁-C₄-Alkyl-, Trifluormethyl-, C₁-C₄-Alkoxy-, Hydroxy-, Amino-, Monomethylamino-, Dimethylamino-, Cyano- oder Nitrogruppen mono- oder disubstituierte Phenyl-, Pyridyl-, Thienyloder Pyrrolgruppe bedeutet und
R² ein Wasserstoffatom oder eine gegebenenfalls durch Fluor, Methoxy, Hydroxy oder Amino substituierte Phenylgruppe ist.
Verfahren zur Herstellung der 3-Azabicyclo[3.2.0]heptan-Derivate der Formel I gemäß Anspruch 1, dadurch gekennzeichnet, daß man ein Amin der Formel II 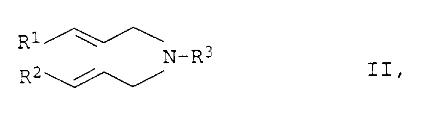 worin R¹ und R² die in Anspruch 1 angegebenen Bedeutungen besitzen und R³ Wasserstoff, Acetyl, Benzyl oder Trifluoraceryl bedeutet, in einem inerten Lösungsmittel bei einer Temperatur von 20-80°C in Gegenwart von etwa 1 Mol Salzsäure prc Mol Amin photochemisch einer 2+2 Cycloaddition unterwirft und eine gegebenenfalls vorhandene Acyl- oder Benzylgruppe für R³ abspaltet.

## Claims

1. A 3-azabicyclo[3.2·0]heptane derivative of the formula I 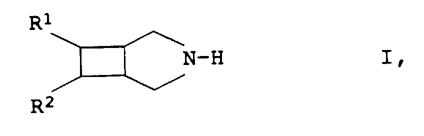 where
R¹ is phenyl, pyridyl, thienyl or pyrrolyl which is unsubstituted or mono- or disubstituted by halogen atoms, C₁-C₄-alkyl, trifluoromethyl, C₁-C₄-alkoxy, hydroxyl, amino, monomethylamino, dimethylamino, cyano or nitro groups, and
R² is hydrogen or phenyl which is unsubstituted or substituted by fluorine, methoxy, hydroxyl or amino.
A process for preparing a 3-azabicyclo[3.2.0]heptane derivative of the formula I as claimed in claim 1, which comprises subjecting an amine of the formula II 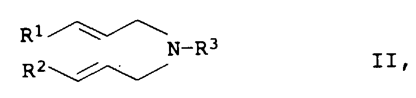 where R¹ and R² have the meanings specified in claim 1, and R³ is hydrogen, acetyl, benzyl trifluoroacetyl, to a photochemical 2+2 cycloaddition in an inert solvent at 20-80°C in the presence of about 1 mole of hydrochloric acid per mole of amine, and eliminating any acyl or benzyl group R³.

## Revendications

1. Dérivés du 3-azabicyclo[3.2.0]heptane de la formule I 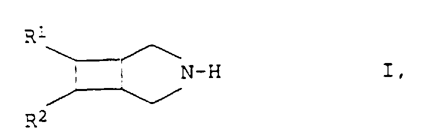 dans laquelle
R¹ représente des radicaux phényle, pyridyle, thiényle, ou pyrrolyle, éventuellement monosubstitués ou disubstitués par des atomes d'halogènes, des radicaux alkyle en C₁ à C₄, trifluorométhyle, alcoxy en C₁ à C₄, hydroxyle, amino, monométhylamino, diméthylamino, cyano, ou nitro et
R² représente un atome d'hydrogène, ou un radical phényle éventuellement substitué par du fluor, un groupe méthoxy, hydroxy ou amino.
2. Procédé de préparation des dérivés du 3-azabicyclo[3.2.0]heptane de la formule I suivant la revendication 1, caractérisé en ce que l'on soumet une amine de la formule II 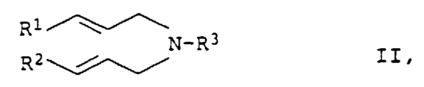 dans laquelle R¹ et R² possèdent les significations qui leur ont été attribuées dans la revendication 1 et R³ représente un atome d'hydrogène, un radical acétyle, benzyle, ou trifluoracétyle, dans un solvant inerte, à une température de 20 à 80°C et en présence d'environ 1 mole d'acide chlorhydrique par mole d'amine par voie photochimique à une cycloaddition 2+2 et on sépare le groupe acyle ou benzyle éventuellement présent pour R³.
